# EUROPEAN PATENT SPECIFICATION

(11) **EP 2 729 452 B1**
(45) Date of publication and mention of the grant of the patent: **20.03.2019**
(21) Application number: 12807882.1
(22) Date of filing: 03.07.2012
(51) Int. Cl.: C07D 233/58, C07F 3/06, B01J 31/18, B01J 20/22, B01J 20/30, B01J 31/16

(54) **PROCESS FOR PREPARING POROUS METAL-ORGANIC FRAMEWORK COMPOSED OF ZINC METHYLIMIDAZOLATE**
VERFAHREN ZUR HERSTELLUNG EINES PORÖSEN METALLORGANISCHEN RAHMENS AUS ZINK- METHYLIMIDAZOLAT
PROCÉDÉ DE PRÉPARATION D'UNE STRUCTURE POREUSE MÉTAL-COMPOSÉ ORGANIQUE À BASE DE MÉTHYLIMIDAZOLATE DE ZINC

(30) Priority: 06.07.2011 EP 11172802
(43) Date of publication of application: 14.05.2014
(73) Proprietor: BASF SE, 67056 Ludwigshafen am Rhein (DE)
(72) Inventor: TRUKHAN, Natalia, 67071 Ludwigshafen (DE); MÜLLER, Ulrich, 67435 Neustadt (DE)
(74) Representative: BASF IP Association
(86) International application number: PCT/IB2012/053368
(87) International publication number: WO 2013/005160

(56) References cited:
- WO-A1-01/46290
- WO-A1-2010/058123
- WO-A2-2007/087434
- US-A- 3 792 016
- US-A1- 2010 160 661
- JOHN E. BAUMAN ET AL: "Imidazole Complexes of Nickel(II), Copper(II), Zinc(II), and Silver(I)", INORGANIC CHEMISTRY, vol. 3, no. 3, 1 March 1964 (1964-03-01), pages 368-373, XP055185338, ISSN: 0020-1669, DOI: 10.1021/ic50013a014
- HUANG X. C. ET AL.: 'Ligand-Directed Strategy for Zeolite-Type Metal-Organic Frameworks: Zinc(II) Imidazolates with Unusual Zeolitic Topologies' ANGEW. CHEM. vol. 118, 27 January 2006, pages 1587 - 1589, XP002500484
- PARK K. S. ET AL.: 'Exceptional chemical and thermal stability of zeolitic imidazolate frameworks' PROC. NAT. ACAD. SCI. U.S.A. vol. 103, no. 27, 05 July 2006, pages 10186 - 10191, XP002667019

## Description

The present invention relates to a process for preparing a porous metal-organic framework composed of zinc 2- methylimidazolate.

Porous metal-organic frameworks are known in the prior art and form an interesting class of substances which can be an alternative to zeolites for various applications. Numerous processes have been developed for preparing such porous metal-organic frameworks. Typically, a metal salt is reacted with the at least bidentate organic compound, for example a dicarboxylic acid, in a suitable solvent under superatmospheric pressure and at elevated temperature.

These preparative processes frequently do not provide a basis for the production of relatively large amounts.

Preparative processes of this type are also known for the zinc 2-methylimidazolate known in the prior art as metal-organic framework.

WO 2007/131955 A1 describes, for example, the electrochemical preparation of this framework. Here, zinc is made available to the reaction space by anodic oxidation. Despite the very good yields, this process is expensive and has only limited suitability for industrial production.

J. Cravillon et al., Chem. Mater. 21 (2009), 1410-1412, describe the preparation of the framework in only moderate yields in methanol and N,N-dimethylformamide (supporting information).

X.-C. Huang et al., Angew. Chem. 118 (2006), 1587-1589, describe the preparation of the framework, which is concluded only after one month and gives moderate yields.

US 3 792 015 A and WO 01/46290 A1 disclose preparation of zinc imidazolate MOF starting from different zinc precursors and using water as solvent during the reaction of the precursors.

Despite the preparative processes known in the prior art, there is a need for new processes in which the disadvantages of the prior art are at least partly overcome and, in particular, allow preparation of the framework in relatively large amounts, in particular in very good absolute yields (based on a starting material) and yields on a time basis (space-time yield).

It is an object of the present invention to provide such a process.

The object is achieved by a process for preparing a porous metal-organic framework comprising at least one at least bidentate organic compound coordinated to at least one metal ion, where the at least one metal ion is a zinc ion and the at least one at least bidentate organic compound is based on 2-methylimidazole, which comprises the steps
(a) addition of a first water-based solution comprising zinc ions to a second water-based solution comprising 2-methylimidazole, with a suspension being formed after addition of the second solution;
(b) addition of a third solution comprising a strong base to the suspension formed in step (a),
wherein the first water-based solution is a zinc sulfate solution, wherein the first, the second or the first and the second water-based solution is a methanol/water solution, wherein the third solution is a water-based alkali metal hydroxide solution, and wherein the molar ratio of zinc to alkali metal hydroxide after the end of the addition in step (b) is in the range from 1:5 to 1:1.

It has surprisingly been found that high space-time yields can be achieved when the abovementioned features of the process of the invention are adhered to. In particular, it is surprising that the framework obtained can be obtained not only virtually quantitatively but also with very good specific surface areas.

The porous metal-organic framework prepared by the process of the invention comprises at least one metal ion which is a zinc ion. However, it is likewise possible for more than one metal ion to be present in the porous metal-organic framework. These one or more metal ions other than zinc can be located in the pores of the metal-organic framework or participate in formation of the lattice of the framework. In the latter case, such a metal ion would likewise bind the at least one at least bidentate organic compound or a further at least bidentate organic compound.

Possible metal ions here are in principle all metal ions which are suitable as part of the porous metal-organic framework. If the porous metal-organic framework comprises more than one metal ion, these metal ions can be present in a stoichiometric or nonstoichiometric amount. If coordination sites are occupied by a further metal ion and this is present in a nonstoichiometric ratio to the abovementioned metal ion, such a porous metal-organic framework can be considered to be a doped framework. The preparation of such doped metal-organic frameworks in general is described in EP-A 1 785 428.

The porous metal-organic framework has only one metal ion (zinc ion).

In addition, the porous metal-organic framework can, after the reaction according to the process of the invention, be impregnated with a further metal in the form of a metal salt. An impregnation process is described, for example, in EP-A 1070538.

If a further metal ion is present in a stoichiometric ratio to zinc, mixed metal frameworks are obtained. Here, the further metal ion can participate in the construction of the framework or not participate.

The framework is preferably made up of only zinc ions and the at least one at least bidentate organic compound.

In addition, the porous metal-organic framework comprises at least one at least bidentate organic compound based on 2-methylimidazole.

For the purposes of the present invention, the term "based" refers to 2-methylimidazole or its anion, preferably only its anion.

The metal-organic framework can also have one or more further at least bidentate organic compounds which are different from 2-methylimidazole.

For example, the framework can comprise nitrogen-based organic compounds as are described in WO 2007/131955 A1.

Accordingly, the at least one further organic compound can be a monocyclic, bicyclic or polycyclic ring system which is derived from at least one heterocycle selected from the group consisting of pyrrole, alpha-pyridone and gamma-pyridone and has at least two ring nitrogens, where the ring system is unsubstituted or has one or more substituents selected independently from the group consisting of halogen, C₁₋₆-alkyl, phenyl, NH₂, NH(C₁₋₆-alkyl), N(C₁₋₆-alkyl)₂, OH, Ophenyl and OC₁₋₆-alkyl, where the substituents C₁₋₆-alkyl and phenyl are unsubstituted or have one or more substituents selected independently from the group consisting of halogen, NH₂, NH(C₁₋₆-alkyl), N(C₁₋₆-alkyl)₂, OH, Ophenyl and OC₁₋₆-alkyl, with the exception of 2-methylimidazole.

For the purposes of the present invention, the term "C₁₋₆-alkyl" refers to an alkyl group having from 1 to 6 carbon atoms. Examples are methyl, ethyl, n-propyl, i-propyl, n-butyl, i-butyl, sec-butyl, t-butyl, pentyl, hexyl. Preferred radicals are methyl and ethyl. If a substituted C₁₋₆-alkyl radical is present, at least one hydrogen atom is replaced by another substituent.

Furthermore, for the purposes of the present invention, the term "halogen" refers to fluorine, chlorine, bromine or iodine. Preference is given to fluorine and chlorine.

As indicated above, the further organic compound can be a monocyclic, bicyclic or polycyclic ring system which is derived at least from one of the heterocycles selected from the group consisting of pyrrole, alpha-pyridone and gamma-pyridone. All these three heterocycles have a ring nitrogen bearing a hydrogen which can be eliminated in at least one mesomeric structure. It is thus possible for the pyrrole, alpha-pyridone or gamma-pyridone to be deprotonated. This forms a negative charge which can at least partially compensate the positive charge of the at least one metal ion.

For the purposes of the present invention, the term "derive" means that the monocyclic, bicyclic or polycyclic ring system has at least one substructure which corresponds to pyrrole, alpha-pyridone or gamma-pyridone. In addition, two or all three heterocycles can also be present as substructure in the ring system.

For the purposes of the present invention, the term "derive" also means that the three abovementioned heterocycles do not have to be present in neutral form but can optionally also occur as anion or cation, so that the oxidation can also be carried out in the presence of these ions.

In addition, it has to be taken into account that at least one of the heterocycles representing a substructure of the ring system is deprotonated during the reaction.

Furthermore, the term "derive" means, for the purposes of the present invention, that the substructure of at least one of the three heterocycles can have substituents and one or more ring carbons can be replaced by a heteroatom.

Of course, the ring system can also be one of the heterocycles pyrrole, alpha-pyridone or gamma-pyridone itself or the ring system can likewise be made up of substructures which are selected exclusively from the group consisting of pyrrole, alpha-pyridone and gamma-pyridone. In this case, too, the above-described modifications are possible.

Finally, it has to be taken into account that at least one hydrogen which in at least one mesomeric structure is not the hydrogen bound to said nitrogen is replaced by a bond by means of which the corresponding heterocycle is bound to the remainder of the ring system.

If the ring system is a monocyclic ring system, this is derived from pyrrole or alpha-pyridone or gamma-pyridone.

However, the ring system can also be a bicyclic ring system. This is the case when, for example, two rings which are joined to one another via a single covalent bond or via a group R are present in the ring system. Here, one ring has to be derived from pyrrole, alpha-pyridone or gamma-pyridone.

R can be -O-, -NH-, -S-, -N=N- or an aliphatic branched or unbranched saturated or unsaturated hydrocarbon having from 1 to 4 carbon atoms which may be interrupted by one or more independent atoms or functional groups selected from the group consisting of -O-, -NH-, -S- and -N=N-.

Furthermore, the bicyclic ring system can be a fused ring system.

Examples are, in particular, benzofused derivatives derived from pyrrole, alpha-pyridone and gamma-pyridone.

In addition, the bicyclic ring system can be a bridged ring system.

The ring system can likewise be a polycyclic ring system having, for example, 3, 4 or more rings. Here, the rings can be joined via a single covalent bond and/or a group R and/or be present in fused form and/or as bridged ring system.

The ring system has at least two ring nitrogens. At least one of the two ring nitrogens is the nitrogen present in the ring which is derived from pyrrole, alpha-pyridone and gamma-pyridone. In addition, at least one further ring nitrogen has to be present. If the ring system is a system having more than one ring, the at least second ring nitrogen can be present in the ring derived from pyrrole, alpha-pyridone or gamma-pyridone or, if the at least one further ring is not derived from these three heterocycles, in this ring.

Preference is given to the at least two ring nitrogens being present in one ring of the ring system.

In this case, the ring is derived from pyrazole, imidazole (with the exception of 2-methylimidazole), pyridazin-2-one and pyrimidin-2-one or pyrimidin-4-one.

In addition to the two ring nitrogens, further ring nitrogens can be present. For example, the ring system can have 3, 4, 5 or more ring nitrogens.

If more than two ring nitrogens are present, all ring nitrogens can be present in one ring of the ring system or be distributed over more than one ring up to all rings of the ring system.

When, for example, three ring nitrogens are present, these are preferably present in the ring which is derived from pyrrole, alpha-pyridone or gamma-pyridone. The resulting substructure of the ring can, for example, then be derived from a triazole such as 1,2,3-triazole or 1,2,4-triazole.

In addition, the ring system can have further heteroatoms in the ring. These can be, for example, oxygen or sulfur. However, preference is given to no further heteroatoms apart from nitrogen being present.

If the ring system has more than one ring, this ring can be saturated or unsaturated. The at least one further ring preferably has an at least partially conjugated double bond system or is aromatic in nature.

The ring system can be unsubstituted.

Furthermore, the ring system can have one or more substituents. If a plurality of substituents are present, these can be identical or different.

The substituents bound to the ring system can be halogen, C₁₋₆-alkyl, phenyl, NH₂, NH(C₁₋₆-alkyl), N(C₁₋₆-alkyl)₂, OH, Ophenyl or OC₁₋₆-alkyl.

If at least one of the abovementioned substituents on the ring system is a C₁₋₆-alkyl or phenyl, these can likewise be unsubstituted or have one or more substituents. Here too, when a plurality of substituents are present, it is possible for these to be identical or different. These are selected from the group consisting of halogen, NH₂, NH(C₁₋₆-alkyl), N(C₁₋₆-alkyl), N(C₁₋₆-alkyl)₂, OH, Ophenyl and OC₁₋₆-alkyl.

If the C₁₋₆-alkyl group occurs more than once, these alkyl groups can be identical or different.

For the purposes of the present invention, the hydroxy or keto group of the alpha-pyridone or gamma-pyridone is not considered to be a substituent since this group is necessary in the ring in order to obtain a ring nitrogen to which hydrogen is bound for at least one mesomeric structure.

The substituents which are bound to the ring system preferably do not have any further substituents.

Preferred substituents which are bound to the ring system are C₁₋₆-alkyl, phenyl, NH₂ and OH. Further preference is given to C₁₋₆-alkyl and NH₂. Particular preference is given to C₁₋₆-alkyl.

In a further preferred embodiment, the ring system is selected from the group consisting of

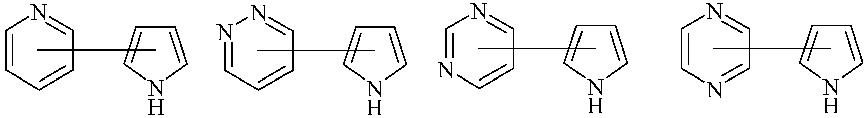

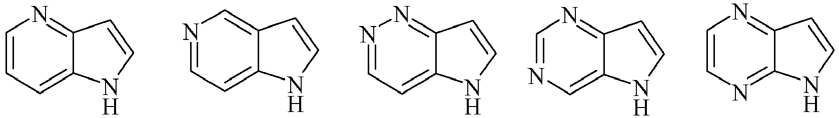

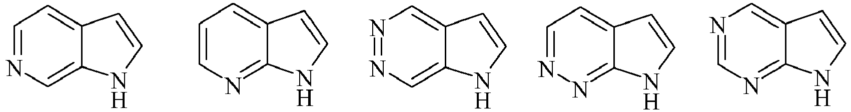

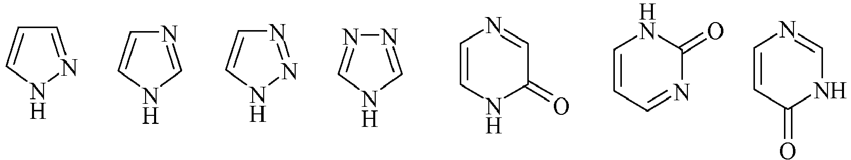

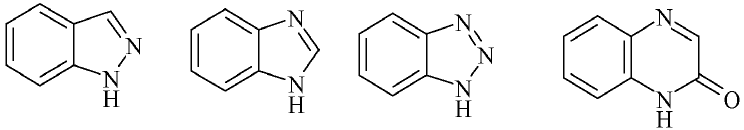

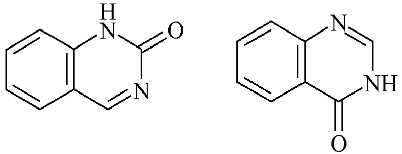

The ring system is more preferably an imidazole (with the exception of 2-methylimidazole), benzimidazole, triazole, 2-hydroxypyrimidine or 4-hydroxypyrimidine.

The at least one organic compound is very particularly preferably selected from the group consisting of 2-ethylimidazole, benzimidazole, 1,2,4-triazole, 3-amino-1,2,4-triazole, 3,5-diamino-1,2,4-triazole, 2-hydroxypyrimidine and 4-hydroxypyrimidine and protonated forms thereof.

However, the porous metal-organic framework preferably has only one at least bidentate organic compound (2-methylimidazol(at)e).

Particular preference is given to a porous metal-organic framework made up of Zn(ll) ions to which 2-methylimidazolate is coordinated to form a framework structure.

In step (a) of the process of the invention, a first water-based solution comprising zinc ions is added to a second water-based solution comprising 2-methylimidazole, with a suspension being formed after addition of the second solution.

The first solution is a water-based solution comprising zinc ions. Accordingly, the first solution has a solvent or solvent mixture in which at least zinc ions are present in solution. For the purposes of the present invention, "water-based" means that the solvent is a mixture comprising at least 40% by weight (preferably at least 50% by weight, more preferably more than 50% by weight) of water, based on the total amount of solvent, is present.

The first solution comprises zinc ions which are typically present in the form of a dissolved zinc compound. The first solution is a zinc sulfate solution, preferably in the form of its heptahydrate.

The second solution is a water-based solution comprising 2-methylimidazole. Accordingly, the second solution has a solvent or solvent mixture in which at least 2-methylimidazole is present in solution. For the purposes of the present invention, "water-based" means that the solvent is a mixture comprising not more than 70% by weight (preferably not more than 60% by weight, more preferably not more than 50% by weight, more preferably not more than 40% by weight, more preferably less than 40% by weight) of water, based on the total amount of solvent, is present. The second solution comprises 2-methylimidazole. In addition, the second solution can comprise further materials such as one or more further at least bidentate organic compounds. The second solution preferably comprises only 2-methylimidazole.

The molar ratio of zinc ions added to 2-methylimidazole after the end of the addition in step (a) is preferably in the range from 1:5 to 1:1. Greater preference is given to a range from 1:4 to 1:1.1, more preferably from 1:3 to 1:1.5, even more preferably from 1:2.5 to 1:1.75, in particular 1:2.

The first, the second or the first and the second water-based solution is a methanol/water solution. The proportion by weight of methanol in the first solution is preferably not more than 60% by weight, based on the total amount of the mixture, more preferably not more than 50% by weight, more preferably less than 50% by weight. In the second solution, the proportion can be at least 30% by weight, preferably at least 40% by weight, more preferably at least 50% by weight, more preferably at least 60% by weight, preferably at least 70% by weight.

Addition of the second solution forms a suspension. It will be clear to a person skilled in the art that the commencement of suspension formation is dependent on various factors and thus occurs right at the beginning of addition, between the additions, if the addition is carried out discontinuously, or after the addition is finally complete. Preference is given to continuous addition. The suspension comprises framework formed.

In step (b) of the process of the invention, a third solution which is alkaline is added to the suspension formed in step (a).

Step (b) thus commences when a suspension has been formed in step (a). Steps (a) and (b) can thus occur partially synchronously. Preference is given to step (b) commencing only when step (a) is concluded.

The third solution is alkaline. This is achieved by using a strong base in a solvent or solvent mixture. It is also possible to use a plurality of bases.

The term "strong base" preferably refers to a Brønsted base which gives a corresponding aqueous solution having a pH given by the equation pH = 14 + Ig c_{Base}.

As base, it is used an alkali metal hydroxide or a mixture of a plurality of different alkali metal hydroxides. Examples are, in particular, sodium hydroxide and potassium hydroxide. The solvent is a water-based mixture (water-based solution). The proportion of water in this mixture is preferably more than 50% by weight. The third solution is preferably an aqueous solution (100% water as solvent). In particular, a water-based (in particular aqueous) alkali metal hydroxide (in particular NaOH) solution is preferred.

The addition in steps (a) and (b) can be carried out by known methods. Mention may here be made of, for example, dropwise addition, pouring-in or pumping.

The molar proportion of zinc based on the total amount of the suspension before step (b) is preferably in the range from 0.2 to 0.7 mmol of zinc/g of suspension; preferably in the range from 0.35 to 0.55 mmol of zinc/g of suspension.

The molar proportion of zinc based on the total amount of the suspension after step (b) is preferably in the range from 0.1 to 0.5 mmol of zinc/g of suspension; preferably in the range from 0.2 to 0.4 (more preferably in the range from 0.2 to < 0.4) mmol of zinc/g of suspension.

The molar ratio of zinc to alkali metal hydroxide after the end of the addition in step (b) is preferably in the range from 1:5 to 1:1. Greater preference is given to a range from 1:4 to 1:1.1, more preferably from 1:3 to 1:1.5, even more preferably from 1:2.5 to 1:1.75, in particular 1:2.

The molar ratio of zinc to methanol after the end of the addition in step (b) is preferably in the range from 1:5 to 1:50. Greater preference is given to a range from 1:10 to 1:40, more preferably from 1:15 to 1:30.

The pH before step (b) is preferably below 6.5.

The pH after step (b) is preferably greater than 6.0.

Preference is given to at least one of the steps (a) and (b) being carried out at a temperature in the range from 10°C to 30°C; more preferably in the range from 15°C to 25°C, in particular at from 18°C to 23°C; preferably at room temperature. The steps (a) and (b) are preferably carried out at the same temperature.

Preference is given to carrying out at least one of the steps (a) and (b) at an absolute pressure of not more than 2 bar. However, the pressure is more preferably not more than 1230 mbar (absolute). The reaction particularly preferably takes place at atmospheric pressure. However, slightly superatmospheric or subatmospheric pressures can occur due to the apparatus. For this reason, the term "atmospheric pressure" refers, for the purposes of the present invention, to the pressure range given by the actual atmospheric pressure ± 150 mbar. The steps (a) and (b) are preferably carried out at the same pressure.

Preference is given to at least one of the steps (a) and (b) being carried out with mixing of the suspension, in particular both steps. Mixing can be effected by conventional methods. For example, stirring, shaking, circulation or pumped circulation are conceivable here.

Step (b) is preferably followed by isolation of the metal-organic framework (step c). This can be effected by conventional techniques such as filtration, centrifugation or the like. The isolation in step (c) is preferably carried out by filtration with optional subsequent washing.

The porous metal-organic framework obtained can be subjected to drying. Spray drying is also possible.

Accordingly, process step (c) is followed by a drying step which is preferably carried out after spray drying, should the latter be carried out. The temperature set in drying (with or without spray drying) is typically greater than 60°C, preferably from 80°C to 200°C, more preferably from 100°C to 150°C.

The solids yield of product (framework) is preferably in the range from 2 to 15% by weight. Greater preference is given to a range from 4 to 10% by weight, more preferably from 5 to 8% by weight. Here, the solids yield is the ratio of the amount of dry framework to the total amount after the end of step (b) (weight of framework/sum of the weight of the 1st to 3rd solutions).

### Examples

### Example 1: Synthesis of Zn-2-methylimidazolate ZIF in MeOH/H₂O solution at RT

### Synthesis conditions:

MeOH/H₂O; RT; 1 bar (atmospheric pressure);
Zn:2-mlm:NaOH:MeOH:H₂O = 1:2:2:25:111

### Experimental method:

| Starting material | Molar | Calculated | Experimental |
|---|---|---|---|
| 1) 2-methylimidazole (2-mlm) | 0.2 mol | 16.4 g | 16.4 g |
| 2) zinc sulfate*7 water | 0.1 mol | 28.8 g | 28.8 g |
| 3) sodium hydroxide (NaOH) | 0.2 mol | 8.0 g | 8.0 g |
| 4) methanol (MeOH) | 2.5 mol | 80.0 g | 80.0 g |
| 5) water (H₂O) | 11.1 mol | 200.0 g | 200.0 g |

### Preparation of solution 1:

In a glass beaker, zinc sulfate is dissolved in 50 g of water and 40 g of methanol (pH = 5.2).

### Preparation of solution 2:

In a second glass beaker, 2-methylimidazole is dissolved in 50 g of water and admixed with 40 g of methanol (pH=11.2).

### Preparation of solution 3:

In a third glass beaker, sodium hydroxide is dissolved in 100 g of water (pH = 12.8).

The first solution is added dropwise to the second solution over a period of 60 minutes. As time goes on, a white suspension is formed and the pH drops to 6.38. The sodium hydroxide solution is added dropwise to the white suspension over a period of one hour (final pH = 7.12). The solid is then filtered off and washed twice with 100 ml of water. The filtercake is dried overnight at 150°C in a vacuum drying oven.
Weight of product, moist: 56.90 g
Weight of product after drying: 22.46 g
Solids yield of product: 6.74% by weight
Yield based on Zn: 96.9 mol%
Space-time yield (STY): 766 kg/m³/day

### Analyses:

Langmuir surface area of product: 1725 m²/g (BET analysis: 1284 m²/g)
Chemical analysis: Zn 28.2% by weight, N 24% by weight, S 0.62% by weight.

### Example 2: Synthesis of Zn-2-methylimidazolate ZIF in MeOH/H₂O solution at RT

### Synthesis conditions:

MeOH/H₂O; RT; 1 bar (atmospheric pressure);
Zn:2-mlm:NaOH:MeOH:H₂O = 1:2:2:25:111

### Experimental method:

| Starting material | Molar | Calculated | Experimental |
|---|---|---|---|
| 1) 2-methylimidazole (2-mlm) | 0.2 mol | 16.4 g | 16.4 g |
| 2) zinc sulfate*7 water | 0.1 mol | 28.8 g | 28.8 g |
| 3) sodium hydroxide (NaOH) | 0.2 mol | 8.0 g | 8.0 g |
| 4) methanol (MeOH) | 2.5 mol | 80.0 g | 80.0 g |
| 5) water (H₂O) | 11.1 mol | 200.0 g | 200.0 g |

### Preparation of solution 1:

In a glass beaker, zinc sulfate is dissolved in 50 g of water (pH = 5.1).

### Preparation of solution 2:

In a second glass beaker, 2-methylimidazole is dissolved in 50 g of water and admixed with 80 g of methanol (pH = 9.5).

### Preparation of solution 3:

In a third glass beaker, sodium hydroxide is dissolved in 100 g of water (pH = 12.8).

The first solution is added dropwise to the second solution over a period of 60 minutes. As time goes on, a white suspension is formed and the pH drops to 6.1. The sodium hydroxide solution is added dropwise to the white suspension over a period of one hour (final pH = 11.2). The solid is then filtered off and washed 10 times with 100 ml of water. The filtercake is dried at 150°C in a vacuum drying oven for 48 hours.
Weight of product, moist: 58.57 g
Weight of product after drying: 22.20 g
Solids yield of product: 6.66% by weight
Yield based on Zn: 96.7 mol%
Space-time yield (STY): 758 kg/m³/day

### Analyses:

Langmuir surface area of product: 2091 m²/g (BET analysis: 1533 m²/g)
Chemical analysis: Zn 28.3% by weight, N 24.4% by weight, S 0.18% by weight

### Example 3: Synthesis of Zn-2-methylimidazolate ZIF in MeOH/H₂O solution at RT

### Synthesis conditions:

MeOH/H₂O; RT; 1 bar (atmospheric pressure);
Zn:2-mlm:NaOH:MeOH:H₂O = 1:2:2:18.7:111

### Experimental method:

| Starting material | Molar | Calculated | Experimental |
|---|---|---|---|
| 1) 2-methylimidazole (2-mlm) | 0.2 mol | 16.4 g | 16.4 g |
| 2) zinc sulfate*7 water | 0.1 mol | 28.8 g | 28.8 g |
| 3) sodium hydroxide (NaOH) | 0.2 mol | 8.0 g | 8.0 g |
| 4) methanol (MeOH) | 1.87 mol | 60.0 g | 60.0 g |
| 5) water (H₂O) | 11.1 mol | 200.0 g | 200.0 g |

### Preparation of solution 1:

In a glass beaker, zinc sulfate is dissolved in 50 g of water (pH = 5.1).

### Preparation of solution 2:

In a second glass beaker, 2-methylimidazole is dissolved in 50 g of water and admixed with 60 g of methanol (pH = 9.5).

### Preparation of solution 3:

In a third glass beaker, sodium hydroxide is dissolved in 100 g of water (pH = 12.8).

The first solution is added dropwise to the second solution over a period of 60 minutes. As time goes on, a white suspension is formed and the pH drops to 6.2. The sodium hydroxide solution is added dropwise to the white suspension over a period of one hour (final pH = 11.7). The solid is then filtered off and washed 10 times with 100 ml of water. The filtercake is dried at 150°C in a vacuum drying oven for 48 hours.
Weight of product, moist: 46.76 g
Weight of product after drying: 21.89 g
Solids yield of product: 6.99% by weight
Yield based on Zn: 97 mol%
Space-time yield (STY): 804 kg/m³/day

### Analyses:

Langmuir surface area of product: 1715 m²/g (BET analysis: 1258 m²/g)
Chemical analysis: Zn 28.8% by weight, N 23.7% by weight, S 0.54% by weight

## Claims

1. A process for preparing a porous metal-organic framework comprising at least one at least bidentate organic compound coordinated to at least one metal ion, where the at least one metal ion is a zinc ion and the at least one at least bidentate organic compound is based on 2-methylimidazole, which comprises the steps
(a) addition of a first water-based solution comprising zinc ions to a second water-based solution comprising 2-methylimidazole, with a suspension being formed after addition of the second solution;
(b) addition of a third solution comprising a strong base to the suspension formed in step (a),
wherein the first water-based solution is a zinc sulfate solution,
wherein the first, the second or the first and the second water-based solution is a methanol/water solution,
wherein the third solution is a water-based alkali metal hydroxide solution, and wh
erein the molar ratio of zinc to alkali metal hydroxide after the end of the addition in step (b) is in the range from 1:5 to 1:1.

2. The process according to claim 1, wherein the molar ratio of zinc ions added to 2-methylimidazole after the end of the addition in step (a) is in the range from 1:5 to 1:1.

3. The process according to claim 1 or 2, wherein the molar proportion of zinc based on the total amount of the suspension before step (b) is in the range from 0.2 to 0.7 mmol of zinc/g of suspension.

4. The process according to any of claims 1 to 3, wherein the molar proportion of zinc based on the total amount of the suspension after step (b) is in the range from 0.1 to 0.5 mmol of zinc/g of suspension.

5. The process according to any of claims 1 to 4, wherein the molar ratio of zinc to methanol after the end of the addition in step (b) is in the range from 1:5 to 1:50.

6. The process according to any of claims 1 to 5, wherein the pH before step (b) is below 6.5.

7. The process according to any of claims 1 to 6, wherein the pH after step (b) is greater than 6.0.

8. The process according to any of claims 1 to 7, wherein the solids yield of metal-organic framework is in the range from 2 to 15% by weight.

9. The process according to any of claims 1 to 8, wherein at least one of the steps (a) and (b) is carried out at a temperature in the range from 10°C to 30°C.

10. The process according to any of claims 1 to 9, wherein at least one of the steps (a) and (b) is carried out at an absolute pressure of not more than 2 bar.

11. The process according to any of claims 1 to 10, wherein at least one of the steps (a) and (b) is carried out with mixing of the suspension.

12. The process according to any of claims 1 to 11, wherein step (b) is followed by a step
(c) isolation of the metal-organic framework.

13. The process according to any of claims 1 to 12, wherein the isolation in step (c) is carried out by filtration with optional subsequent washing and/or drying.

## Patentansprüche

1. Verfahren zur Herstellung eines porösen metallorganischen Gerüstmaterials, das mindestens eine an ein Metallion koordinativ gebundene, mindestens zweizähnige organische Verbindung enthält, wobei sich bei dem mindestens einen Metallion um ein Zinkion handelt und die mindestens eine mindestens zweizähnige organische Verbindung auf 2-Methylimidazol basiert, mit den folgenden Schritten:
(a) Zugeben einer ersten wasserbasierten Lösung, die Zinkionen enthält, zu einer zweiten wasserbasierten Lösung, die 2-Methylimidazol enthält, wobei nach Zugabe der zweiten Lösung eine Suspension entsteht;
(b) Zugeben einer dritten Lösung, die eine starke Base enthält, zu der in Schritt (a) entstandenen Suspension,
wobei sich bei der ersten wasserbasierten Lösung um eine Zinksulfatlösung handelt,
wobei es sich bei der ersten, der zweiten oder der ersten und der zweiten wasserbasierten Lösung um eine Methanol/Wasser-Lösung handelt,
wobei es sich bei der dritten Lösung um eine wasserbasierte Alkalimetallhydroxidlösung handelt und
wobei das Molverhältnis von Zink zu Alkalimetallhydroxid nach Beendigung der Zugabe in Schritt (b) im Bereich von 1:5 bis 1:1 liegt.

2. Verfahren nach Anspruch 1, wobei das Molverhältnis von zugegebenen Zinkionen zu 2-Methylimidazol nach Beendigung der Zugabe in Schritt (a) im Bereich von 1:5 bis 1:1 liegt.

3. Verfahren nach Anspruch 1 oder 2, wobei der molare Anteil an Zink, bezogen auf die Gesamtmenge der Suspension vor Schritt (b), im Bereich von 0,2 bis 0,7 mmol Zink/g Suspension liegt.

4. Verfahren nach einem der Ansprüche 1 bis 3, wobei der molare Anteil an Zink, bezogen auf die Gesamtmenge der Suspension nach Schritt (b), im Bereich von 0,1 bis 0,5 mmol Zink/g Suspension liegt.

5. Verfahren nach einem der Ansprüche 1 bis 4, wobei das Molverhältnis von Zink zu Methanol nach Beendigung der Zugabe in Schritt (b) im Bereich von 1:5 bis 1:50 liegt.

6. Verfahren nach einem der Ansprüche 1 bis 5, wobei der pH-Wert vor Schritt (b) unter 6,5 liegt.

7. Verfahren nach einem der Ansprüche 1 bis 6, wobei der pH-Wert nach Schritt (b) größer als 6,0 ist.

8. Verfahren nach einem der Ansprüche 1 bis 7, wobei die Feststoffe Ausbeute an metallorganischem Gerüstmaterial im Bereich von 2 bis 15 Gew.-% liegt.

9. Verfahren nach einem der Ansprüche 1 bis 8, wobei mindestens einer der Schritte (a) und (b) bei einer Temperatur im Bereich von 10 °C bis 30 °C durchgeführt wird.

10. Verfahren nach einem der Ansprüche 1 bis 9, wobei mindestens einer der Schritte (a) und (b) bei einem absoluten Druck von nicht mehr als 2 bar durchgeführt wird.

11. Verfahren nach einem der Ansprüche 1 bis 10, wobei mindestens eine der Schritte (a) und (b) unter Durchmischung der Suspension durchgeführt wird.

12. Verfahren nach einem der Ansprüche 1 bis 11, wobei nach Schritt (b) ein Schritt
(c) Isolieren des metallorganischen Gerüstmaterials
erfolgt.

13. Verfahren nach einem der Ansprüche 1 bis 12, wobei die Isolierung in Schritt (c) durch Filtrieren mit fakultativem nachfolgendem Waschen und/oder Trocknen durchgeführt wird.

## Revendications

1. Procédé de préparation d'une charpente métallo-organique poreuse comprenant au moins un composé organique au moins bidenté coordiné à au moins un ion métallique, l'au moins un ion métallique étant un ion zinc et l'au moins un composé organique au moins bidenté étant basé sur le 2-méthylimidazole, qui comprend les étapes
(a) addition d'une première solution à base d'eau comprenant des ions zinc à une deuxième solution à base d'eau comprenant du 2-méthylimidazole, une suspension étant formée après l'addition de la deuxième solution ;
(b) addition d'une troisième solution comprenant une base forte à la suspension formée à l'étape (a),
dans lequel la première solution à base d'eau est une solution de sulfate de zinc,
dans lequel la première, la deuxième, ou la première et la deuxième solution à base d'eau sont des solutions méthanol/eau,
dans lequel la troisième solution est une solution d'hydroxyde de métal alcalin à base d'eau, et
dans lequel le rapport molaire entre zinc et hydroxyde de métal alcalin après la fin de l'addition à l'étape (b) se situe dans la gamme de 1:5 à 1:1.

2. Procédé selon la revendication 1, dans lequel le rapport molaire entre ions zinc ajoutés et 2-méthylimidazole après la fin de l'addition à l'étape (a) se situe dans la gamme de 1:5 à 1:1.

3. Procédé selon la revendication 1 ou 2, dans lequel la proportion molaire de zinc rapportée à la quantité totale de la suspension avant l'étape (b) se situe dans la gamme de 0,2 à 0,7 mmol de zinc/g de suspension.

4. Procédé selon l'une quelconque des revendications 1 à 3, dans lequel la proportion molaire de zinc rapportée à la quantité totale de la suspension après l'étape (b) se situe dans la gamme de 0,1 à 0,5 mmol de zinc/g de suspension.

5. Procédé selon l'une quelconque des revendications 1 à 4, dans lequel le rapport molaire entre zinc et méthanol après la fin de l'addition à l'étape (b) se situe dans la gamme de 1:5 à 1:50.

6. Procédé selon l'une quelconque des revendications 1 à 5, dans lequel le pH avant l'étape (b) est inférieur à 6,5.

7. Procédé selon l'une quelconque des revendications 1 à 6, dans lequel le pH après l'étape (b) est supérieur à 6,0.

8. Procédé selon l'une quelconque des revendications 1 à 7, dans lequel le rendement en matières solides de la charpente métallo-organique se situe dans la gamme de 2 à 15 % en poids.

9. Procédé selon l'une quelconque des revendications 1 à 8, dans lequel au moins une des étapes (a) et (b) est réalisée à une température dans la gamme de 10 °C à 30 °C.

10. Procédé selon l'une quelconque des revendications 1 à 9, dans lequel au moins une des étapes (a) et (b) est réalisée à une pression absolue ne dépassant pas 2 bar.

11. Procédé selon l'une quelconque des revendications 1 à 10, dans lequel au moins une des étapes (a) et (b) est réalisée avec malaxage de la suspension.

12. Procédé selon l'une quelconque des revendications 1 à 11, dans lequel l'étape (b) est suivie d'une étape (c) isolement de la charpente métallo-organique.

13. Procédé selon l'une quelconque des revendications 1 à 12, dans lequel l'isolement à l'étape (c) est réalisé par filtration avec lavage et/ou séchage consécutifs facultatifs.
